# EUROPEAN PATENT SPECIFICATION

(11) **EP 2 908 786 B1**
(45) Date of publication and mention of the grant of the patent: **04.12.2019**
(21) Application number: 13788817.8
(22) Date of filing: 22.10.2013
(51) Int. Cl.: A61F 7/03, A61F 7/12, A61F 7/00

(54) **COMPACT FLUID WARMER**
KOMPAKTER FLÜSSIGKEITSERWÄRMER
RÉCHAUFFEUR DE FLUIDE COMPACT

(30) Priority: 22.10.2012 US 201261716752 P
(43) Date of publication of application: 26.08.2015
(73) Proprietor: Board of Regents, The University of Texas System, Austin, TX 78701 (US)
(72) Inventor: GILL, Brijesh S., Houston, TX 77007 (US); AROOM, Kevin, Houston, TX 77054 (US); COX Jr., Charles, Bellaire, TX 78701 (US)
(74) Representative: WP Thompson
(86) International application number: PCT/US2013/066150
(87) International publication number: WO 2014/066370

(56) References cited:
- EP-A1- 0 514 922
- JP-A- 2001 104 125
- US-A- 2 191 178
- US-A- 4 294 225
- US-A- 5 062 409
- US-A1- 2008 149 118
- US-A1- 2011 061 839
- US-A1- 2012 065 716
- US-B1- 6 289 888

## Description

### FIELD OF THE INVENTION

The present invention relates to a portable apparatus for warming biocompatible fluids for use in the treatment of patients. The invention may be used to warm intravenous fluids for trauma resuscitation or to warm air from a ventilator circuit. A compact nature of the fluid warmer makes it particularly well suited for field applications, such as surgical hospitals near a combat zone.

### BACKGROUND OF THE INVENTION

Hypothermia is quite common in injured patients, including patients experiencing trauma. Hypothermia produces a number of physiologic derangements which worsen the effects of major injury. Several relevant enzyme systems begin to lose efficiency as their ambient temperature falls. For example, the myocardium, which is dependent on the function of membrane-channel type enzymes for normal electrical function, shows a predictable series of atrial followed by ventricular arrhythmias as core temperature falls below 34°C. Cardiac output is further compromised by poor function of intrinsic myocardial components, with bovine myocardium showing a linear decrease in developed tension with decreasing temperature.

Hypothermia also exacerbates hemorrhagic shock in multiple ways. The onset of coagulopathy, which accompanies hypothermia, has been shown to result from malfunction of both clotting factors and platelets. While profound hypothermia may be tolerated by immersion or cardiac surgery patients, the presence of hypothermia in trauma patients predicts significantly higher mortality. Mortality doubles for heterogeneous groups of trauma patients at 34°C, and survival after trauma is very rare when the core temperature falls below 32°C. This effect is greater for more severely injured patients.

The development of hypothermia comes from several factors. Body heat is convectively lost to the environment, and this effect is enhanced by bleeding or the presence of large surface area burns. The body loses both central thermoregulation and peripheral shivering response after traumatic injury. Less heat is produced peripherally as perfusion decreases in shock.

The administration of intravenous fluids is used in trauma resuscitation. The administration of fluid at ambient temperature, however, induces hypothermia. This condition is worse in more severely injured patients, who require the most fluid and have the least ability to tolerate the additional insult of decreased core temperature. Hypothermia and mortality clearly increase after the administration of five liters of crystalloid or five units of packed red blood cells, and the onset of hypothermia increases the incidence of coagulopathy in injured patients, particularly in the presence of acidosis.

As used herein, the phrase "biocompatible fluid" refers to a fluid that is appropriate for infusion into the human body including, but not limited to, normal saline and its less concentrated derivatives, Ringer's lactate, and hypertonic crystalloid solutions; blood and fractions of blood including plasma, platelets, albumin and cryoprecipitate; intravascular volume expanding blood substitutes including hetastarch, polymerized hemoglobin, perfluorocarbons; medications reconstituted with saline or sterile water; and medical gasses including air, oxygen, helium, nitric oxide, and combinations thereof.

Prior art methods of treating hypothermia include direct intravenous fluid warming. The fluid that is warmed may be the blood other biocompatible liquid. Prior art devices used to warm one or more biocompatible fluids for use in the treatment of trauma have used electricity as their heating source. These systems are referred to herein as "biocompatible liquid infusion systems." Electrically heated biocompatible fluid infusion systems have several shortcomings. If the source of electrical energy is alternating current from a central generating station, the unit can then only be used in locations where such alternating current is available. This significantly limits the locations where the units may be used. Locations such as non-industrialized nations or battlefield locations are likely not have readily available sources of alternating current to power such systems. Batteries may be used to generate electrical energy. However, it is believed that sufficient power to heat a single liter of fluid to 20°C within a ten-minute time period would require a rechargeable battery the size and weight of a large laptop computer. In such a case, the weight of the battery would exceed the weight of a liter of saline fluid. The size and weight of such a unit would severely limit its portability. Additionally, the battery would require recharging after each liter of biocompatible fluid is delivered.

Other conventional warming devices may use a venturi fuel-air mixer to mix fuel and air for being introduced into a catalytic combustion chamber. Heat is generated and transferred in a heat exchanger downstream from the combustion chamber, and exhaust gas is liberated at an exhaust port. This type of warming device entrains ambient air into the fuel flow stream to provide passive mixing of air with fuel prior to entry into a catalytic combustion chamber. The nature of the venturi requires a certain linear length of fuel pathway between the point of fuel introduction and the point of fuel-air mixture discharge to allow for complete fuel and air mixing. If the length of the fuel pathway between the point of fuel introduction and the point of fuel-air mixture discharge is insufficient, the mixing of the fuel and air is incomplete and the mixture will not provide satisfactory combustion on the catalyst. The amount of heat generated will be insufficient to warm the heat exchanger and the exhaust gas discharge may contain unwanted carbon monoxide gas due to incomplete oxidation of the fuel component.
US2012/065716 and US2008/149118 refer to prior art relevant for the present invention.

US 4 294 225 A discloses a diver heater system in which high-pressure air from an air supply is admitted through a pressure regulator. Fuel, such as propane, enters the system from a fuel canister. The air and fuel of controlled volumes mix and flow together into a catalytic combustion chamber where combustion is initiated by a spark generator. The heat of combustion is caused to pass through the walls of the combustion chamber, then through thermoelectric modules, converting part of the heat to electricity. The remaining and larger part of the heat passes to a heat exchanger on the opposite side of the thermoelectric module. Circulating water passes through the heat-exchanging chamber, absorbs the heat produced by the combustion reaction and is further pumped to the diver's suit. The electricity generated by the thermoelectric modules is used to power an electric motor driving a circulating pump.

The present invention overcomes the limitations of prior art biocompatible fluid infusion systems by providing a biocompatible liquid infusion system that is not dependent upon electrical energy as a heat source, thereby enhancing portability and utility in field applications. The present invention is sight enough and compact enough to be used in field hospital environments that are remotely located from large central hospitals and from sources of alternating electrical current. The present invention may also be used to warm air delivered to a patient via a ventilation circuit.

### SUMMARY OF THE INVENTION

In accordance with the present invention, an apparatus comprises gas flow chamber on a first side of the apparatus having an air-fuel mixture inlet, a catalyst compartment containing a catalyst member and at least one tortuous combustion products pathway for moving combustion products from the catalyst compartment to an exhaust gas port, a fluid warming chamber on a second side of the apparatus to conductively receive heat generated in the gas flow chamber and having a fluid inlet connectable to a source of fluid, a fluid warming surface and a fluid outlet connectable to a patient, an air-fuel mixing chamber having an air inlet, a fuel port and an air-fuel mixture outlet, a motor-driven air mover having an air intake to receive ambient air and an air outlet disposed to discharge air to the air inlet of the air-fuel mixing chamber, a fuel assembly comprising a fuel storage tank, a valve to receive a stream of fuel from the tank and a fuel port connector coupled to provide fuel from the valve to the fuel port of the air-fuel mixing chamber, a battery to provide electrical current to operate a motor to drive the air mover, and wherein a stream of an air-fuel mixture emerging from the air-fuel mixing chamber enters the catalytic compartment containing the catalyst member and combusts to create a stream of heated combustion products, wherein the combustion products flow through the at least one tortuous pathway to the exhaust port where the combustion products are liberated to the atmosphere, and wherein a stream of fluid from the source of fluid enters the fluid warming chamber through the fluid inlet, is warmed along the warming surface and is removed from the fluid warming chamber through the fluid outlet.

An embodiment of the apparatus may further comprise a catalyst member that comprises one of palladium and platinum. An embodiment of the apparatus may comprise a tank wherein the fuel stored in the tank is a hydrocarbon gas. An embodiment of the apparatus may further comprise a heat exchanger base comprising a metal alloy. An embodiment of the apparatus may comprise a heat exchanger base comprising a conductive material such as stainless steel or, more preferably, aluminum due to its high conductivity and low density. An embodiment of the apparatus may be used to warm a fluid comprising one of blood and intravenous fluid. An embodiment of the apparatus may further comprise a fuel cell configured to receive a flow of fuel gas and to generate an electrical current to operate an electrically-powered motor within the air mover. An embodiment of the apparatus wherein the valve is adjustable to vary a rate of flow of fuel from the storage tank to the air-fuel mixing chamber. An embodiment of the apparatus may comprise a warming surface of the fluid warming chamber with an undulating surface to increase the surface area across which heat can be received from the gas chamber and transferred to the fluid within the fluid warming chamber.

Another embodiment of the apparatus comprises a heat exchanger base having a first side and a second side, a gas chamber cover securable to the first side of the heat exchanger base to form a gas chamber therebetween, the gas chamber having an inlet, a catalyst compartment, a tortuous pathway and an exhaust port, a biocompatible fluid warming chamber cover securable to the second side of the heat exchanger base to form a biocompatible fluid warming chamber therebetween, the biocompatible fluid warming chamber having an inlet connectable to a source of biocompatible fluid, an outlet connectable to a patient, and a fluid warming surface therebetween, an air-fuel mixing chamber having an outlet sealably engaging the inlet to the gas chamber, a catalyst member disposed within the catalyst compartment of the gas chamber, an air mover having an ambient air inlet and an air outlet sealably engaging an air intake of the air-fuel mixing chamber, a storage tank containing a fuel, and a valve connected intermediate the storage tank and a fuel port of the air-fuel mixing chamber, wherein air from the air mover and fuel from the storage tank are mixed in the air-fuel mixing chamber and discharged through the outlet of the air-fuel mixing chamber to the inlet of the gas chamber, wherein an air-fuel mixture in the catalyst compartment combusts in the presence of the catalyst member to produce combustion products and heat, wherein the combustion products move through the tortuous pathway to the exhaust port, and wherein heat transferred from the gas chamber to the fluid warming surface of the biocompatible fluid warming chamber warms a flow of biocompatible fluids flowing through the biocompatible fluid warming chamber. The embodiment of the apparatus may comprise a catalyst member comprising one of palladium and platinum. An embodiment of the apparatus may comprise a tank wherein the fuel stored in the tank is a hydrocarbon. An embodiment of the apparatus may comprise a heat exchanger base comprising a metal alloy. An embodiment of the apparatus may comprise a heat exchanger base comprising aluminum. An embodiment of the apparatus may comprise a fluid warming chamber wherein the fluid warmed in the fluid warming chamber is one of blood and intravenous fluid. An embodiment of the apparatus may comprise a fuel cell configured to receive a flow of fuel gas and to generate an electrical current to operate a motor within the air mover. An embodiment of the apparatus may comprise a valve that is adjustable to vary a rate of flow of fuel from the storage tank to the air-fuel mixing chamber. An embodiment of the apparatus may comprise a warming chamber wherein the warming surface of the fluid warming chamber comprises an undulating surface to increase the surface area across which heat can be received from the gas chamber and transferred to the fluid within the fluid warming chamber.

An embodiment of the apparatus of the present invention may further comprise a control system. For example, the apparatus may further comprise a controller coupled to receive a signal corresponding to an operating set-point input by a user of the apparatus wherein the controller generates and sends a signal to at least one of the motorized fuel valve and the air mover to adjust at least one of the rate of fuel and the rate of air delivered to the air-fuel mixing chamber.

### BRIEF DESCRIPTION OF THE DRAWINGS

FIG. **1** is a perspective view of an embodiment of a biocompatible fluid warming apparatus of the present invention.
FIG. **2** is an exploded perspective view of the biocompatible fluid warming apparatus of FIG. **1****.**
FIG. **3** is another exploded perspective view of the biocompatible fluid warming apparatus of FIG. **1** .
FIG. **4** is a perspective view of a fuel assembly that can be used with the embodiment of the biocompatible fluid warming apparatus of FIGs. **1-3****.**
FIG. **5** is an elevation view of the outlet of an air mover of an embodiment of the apparatus of the present invention.
FIG. **6** illustrates a control system that can be used for an embodiment of the apparatus of the present invention.

### DETAILED DESCRIPTION OF PREFERRED EMBODIMENTS

An embodiment of the biocompatible fluid warming apparatus of the present invention provides a reduced overall volume of the apparatus and a corresponding increased power density in terms of the amount of heat transfer per unit volume. An embodiment of the biocompatible fluid warming apparatus of the present invention provides an improved air-fuel mixing chamber and a tortuous combustion products pathway within the apparatus to promote efficient transfer of heat from the combustion products moving through the combustion products pathway to the fluid to be warmed and introduced into a patient's body.

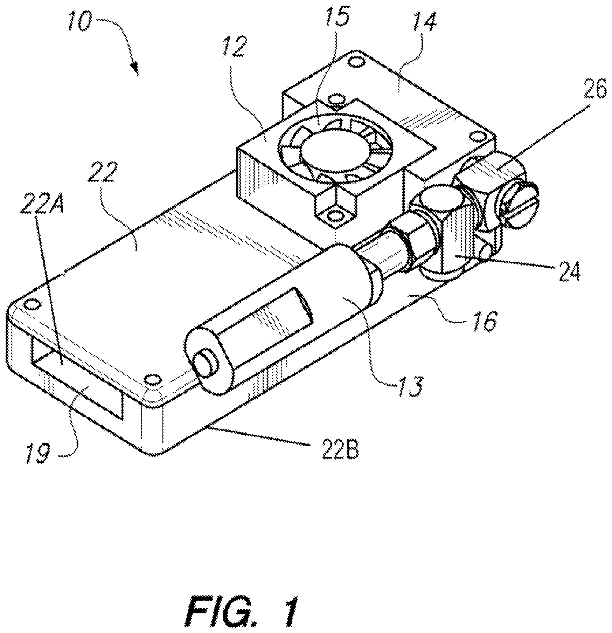

FIG. **1** is a perspective view of a portion **10** of an embodiment of a biocompatible fluid warming apparatus of the present invention. The portion **10** of the apparatus illustrated in FIG. **1** comprises a gas chamber cover **22** coupled to a heat exchanger base **16**, an air mover **12**, an air-fuel mixing chamber **14** coupled to the gas chamber cover **22**, and a fuel assembly **13** coupled to the air-fuel mixing chamber **14.** It should be noted that instead of the space-consuming venturi pathway common in conventional biocompatible fluid warming apparatuses, embodiments of the biocompatible fluid warming apparatus of the present invention include a compact air mover **12** such as, for example, a centrifugal fan, to receive and to move a stream of ambient air to an air-fuel mixing chamber **14.**

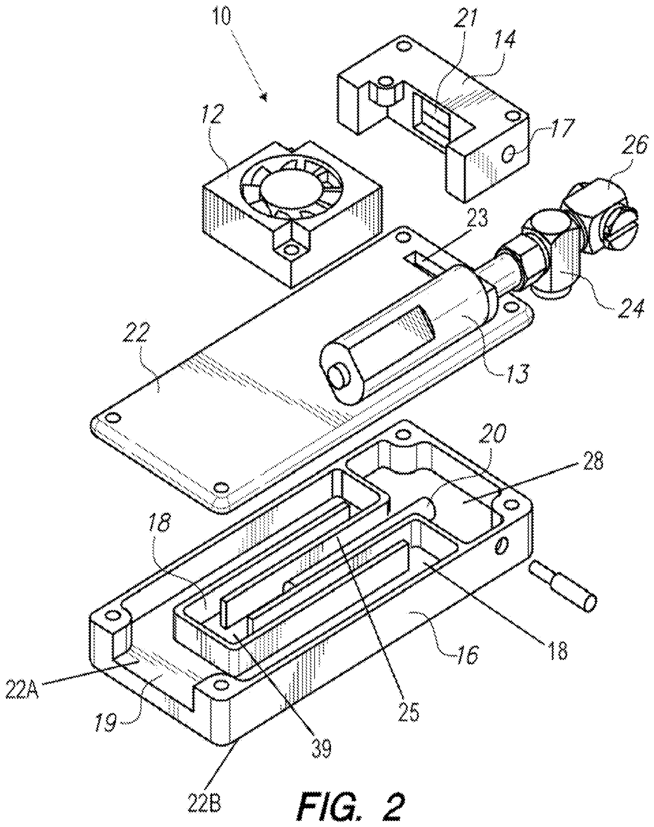

The embodiment of the heat exchanger base **16** illustrated in FIG. **1** and further illustrated in FIG. **2** comprises a gas chamber **39** having a receiving space **28**, a catalyst compartment **25** and two tortuous pathways **18** originating at the catalyst compartment **25** and terminating at an exhaust port **19**. A catalyst member **20** comprising a catalytic material that promotes combustion of the air-fuel mixture is disposed in the catalyst compartment **25** of the gas chamber **39** of the heat exchanger base **16**. The catalyst material may be, for example, palladium or platinum. A pre-warmed air-fuel mixture enters the receiving space **28** of the gas chamber **39** of the heat exchanger base **16** through the gas chamber inlet **23** and moves through the catalyst compartment **25** and along the catalyst member **20**. The catalyst member **20** promotes reaction of the pre-warmed air-fuel mixture to combustion gases to liberate heat. It will be understood that the composition of the combustion products depends on the fuel and will commonly include carbon dioxide and water vapor.

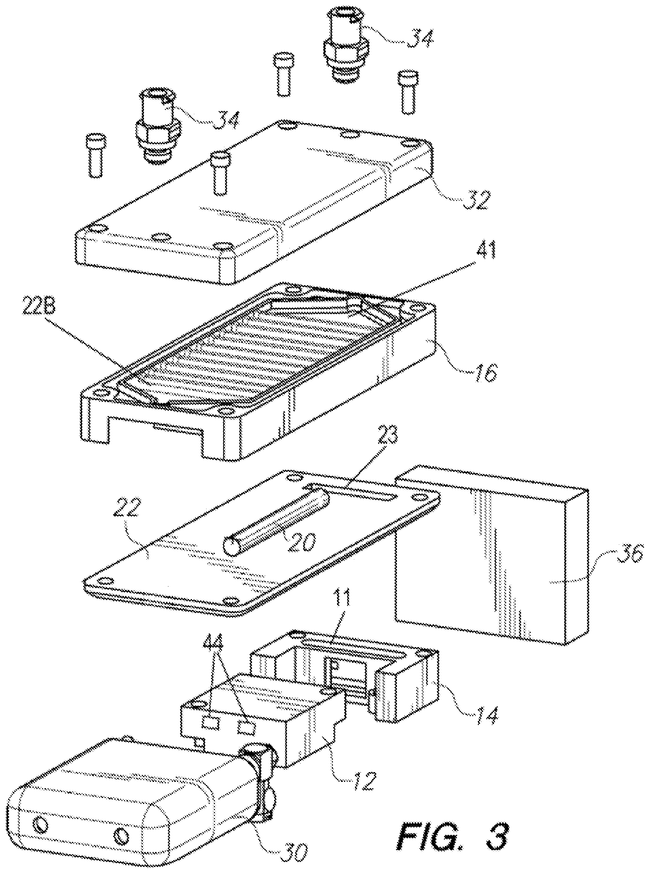

The hot combustion gases created by catalytic combustion of the air-fuel mixture in the catalyst compartment **25** move to and through the tortuous pathways **18** to the exhaust port **19** where they are liberated to the atmosphere. It will be understood that the heat generated by the combustion of the air-fuel mixture is transferred across the heat exchanger base **16** from a first side **22A** (illustrated in FIGs. **1** and **2**) to a second side **22B** (illustrated in FIG. **3**). It will be further understood that the tortuous pathways **18** are adapted to increase residence time of the hot combustion gases within the gas chamber **39** and downstream of the catalyst compartment **25** to thereby increase the amount of heat transferred to a fluid in the fluid warming chamber described in connection with FIG. **3****.**

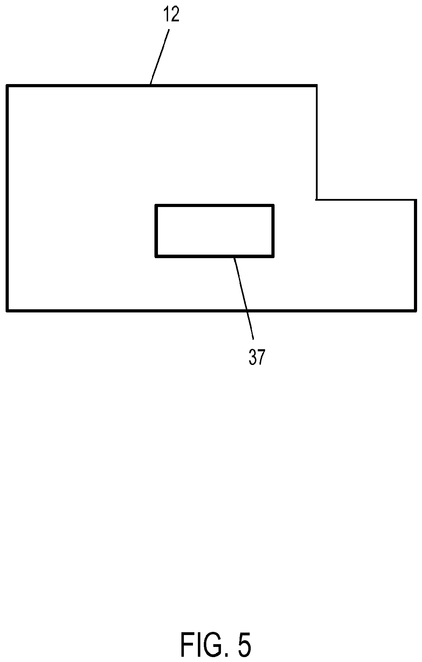

The air mover **12** illustrated in the coupled configuration in FIG. **1** is illustrated as removed from the gas chamber cover **22** in FIG. **2**. The air mover **12** discharges air through an outlet (shown in FIG. **5**) of the air mover **14** to the inlet **21** of the air-fuel mixing chamber **14.** It will be noted that in the assembled view of the portion **10** of the apparatus shown in FIG. **1**, the air mover **12** is coupled to the gas chamber cover **22** immediately adjacent to the air-fuel mixing chamber **14** to sealably engage the outlet (shown in FIG. **5**) of the air mover **12** with the intake **21** of the air-fuel mixing chamber **14** (shown in FIG. **2**). The air-fuel mixing chamber **14** also receives a stream of fuel from a compressed fuel storage tank **13** through a fuel port **17** (shown in FIG. **2**). In the embodiment of FIGs. **1** and **2**, compressed fuel gas or pressurized liquid fuel stored in the tank **13** is controllably throttled and/or released across a motorized needle valve **24** connected intermediate the tank **13** and a precision fuel delivery orifice **26** that is coupled to the fuel port **17** of the air-fuel mixing chamber **14.** It will be understood that, although the embodiment of the apparatus of the present invention illustrated in the appended drawings comprises a motorized needle valve **24**, a manually adjustable needle valve can also be used in alternate embodiments of the apparatus. It will be understood that the air stream and the fuel stream separately introduced into the air-fuel mixing chamber **14** through the air inlet **21** and the fuel port **17**, respectively, are mixed within the air-fuel mixing chamber **14** by movement of the air stream discharged from the air mover **12** and by the movement of the fuel stream as it enters the air-fuel mixing chamber **14.** In the assembled configuration of the portion **10** of the apparatus of FIG. **1**, the air-fuel mixing chamber **14** is coupled to the gas chamber cover **22** proximal to the gas chamber inlet **23** to provide for pre-warming of an air-fuel mixture stream emerging from the outlet **11** (see FIG. **3**) of the air-fuel mixing chamber 1**4.** Pre-warming the air-fuel mixture results in a greater operating temperature in the heat exchanger base **16** and an increased heat exchange efficiency of the apparatus.

As can be seen in FIG. **3**, the air-fuel mixture emerges from the outlet **11** of the air-fuel mixing chamber **14** and enters the gas chamber **39** (not shown in FIG. **3** - see FIG. **2**) between the heat exchanger base **16** and the gas chamber cover **22** through the heat exchanger base inlet **23** of the gas chamber cover **22.** The catalyst member **20**, shown in FIG. **2** in the catalyst compartment **25** and in FIG. **3** removed from the catalyst compartment **25**, is elongate and is resides in the elongate catalyst compartment **25** of the gas chamber **39.** The tortuous combustion product pathways **18** originate at the catalyst compartment **25** and terminate at the exhaust port **19** at an end of the heat exchanger base **16**. The embodiment of the heat exchanger base **16** illustrated in FIG. **2** illustrates a configuration of two separate pathways **18** for moving combustion products from the catalyst compartment **25** to the exhaust port **19**, each pathway **18** having switchbacks to provide for increased residence time within the gas chamber **39** of the hot combustion products gases emerging from the catalyst compartment **25.** The increased residence time results in improved overall heat transfer efficiency. It should be noted that the catalyst member **20** can be selectively positioned proximally (closer to the receiving space **28**) or distally (closer to the exhaust port **19**) within the catalyst compartment **25** for "fine-tuning" of air-fuel mixing occurring upstream of the catalyst compartment **25** and for optimization of the catalytic combustion efficiency.

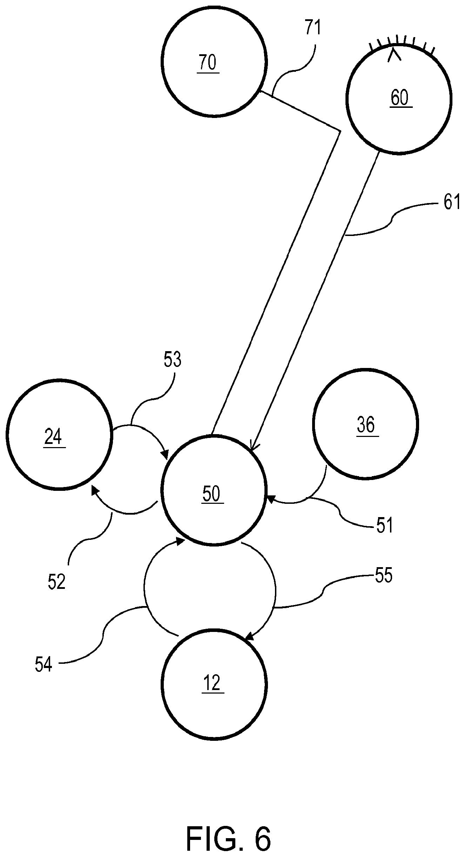

The biocompatible fluid warming apparatus illustrated in the appended drawings includes a generally flat and rectangular heat exchanger base **16**, but this particular design aspect is not crucial to the function. Alternatively, a cylindrical heat exchanger base as disclosed in U.S. Patent 7,261,537 may be used. Alternately, the gas chamber cover **22** may comprise a catalyst to supplement or complement the catalyst member **20.** It will be understood that the motorized needle valve **24** and the precision fuel delivery orifice **26** may be either manually or automatically adjusted and/or modified to optimize the rate of fuel flow to the air-fuel mixing chamber **14.** Control of the operation of apparatus will be discussed in connection with FIG. **6****.**

FIG. **3** illustrates the heat exchanger base **16** and gas chamber cover **22**, air-fuel mixing chamber **14**, air mover **12**, motorized needle valve **24**, precision fuel delivery orifice **26**, fluid warming chamber **41**, fluid warming chamber cover **32** and a pair of Luer lock fittings **34** coupled to the fluid warming chamber cover **32.** A battery pack **36** is provided to supply electrical current to a motor (not shown) incorporated within the air mover **12** or, alternately, a fuel cell **30** engages the air mover **12** through electrical contacts **44** to provide electrical current to operate the air mover **12** and, optionally, to operate the motorized needle valve **24**. It will be understood that a variety of fuels may be stored in the tank **13** and used to fuel the catalytic combustion such as, for example, butane or propane.

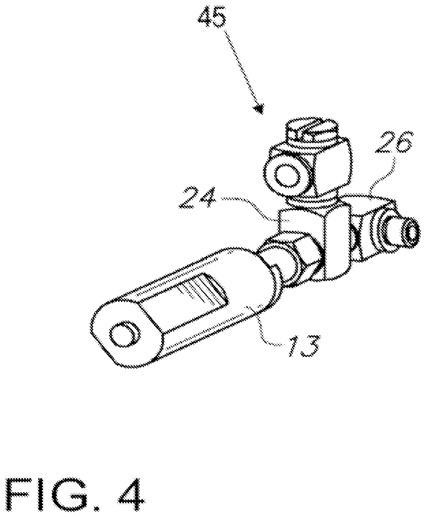

FIG. **4** is a view of an embodiment of the fuel assembly **45** comprising the tank **13**, the motorized needle valve **24** and the precision fuel delivery orifice **26**.

FIG. **5** is an elevation view of the outlet **37** of an air mover **12** of an embodiment of the apparatus of the present invention. The outlet **37** delivers air discharged from the air mover **12** into the inlet **21** of the air-fuel mixing chamber **14.** It will be understood that a seal may be provided about the outlet **37** of the air mover **12** and/or about the inlet **21** of the air-fuel mixing chamber **14.**

FIG. **6** illustrates a control system that can be used for an embodiment of the apparatus of the present invention. The air mover **12** and the motorized needle valve **24** are shown along with the battery **36** as components that interact through a controller **50**. The controller **50** may be electronically coupled to receive an operating set-point signal **61** entered on an input instrument which may comprise a dial, keypad, button, switch, slide, etc. The controller **50** reads the operating set-point signal **61** and compares it to a valve position signal **53** that indicates the amount of fuel being provided to the air-fuel mixing chamber **14** (not shown in FIG. **6**). The controller **50** may automatically adjust the position of the motorized needle valve **24** by generating and sending a signal **52** to the motorized needle valve **24** corresponding to the operating set-point signal **61**. The controller **50** may further generate and send a signal **55**, such as an electrical current, to the air mover **12** to adjust the throughput of the air mover **12** to correspond to the adjusted fuel rate provided by the adjustment of the position of the motorized needle valve **24**,

In an alternate control scheme, the controller **50** reads the operating set-point signal **61** and compares it to an air mover throughput signal **54** that indicates the amount of air being moved through the air mover **12** to burn the fuel being provided to the air-fuel mixing chamber **14** (not shown in FIG. **6**). The controller **50** may adjust the throughput of the air mover **12** by generating and sending a signal **55**, such as an electrical current, to the air mover **12** corresponding to the operating set-point signal **61**. The controller **50** may further generate and send a signal **52** to adjust the position of the motorized needle valve **24** to adjust the rate of fuel delivered to the air-fuel mixing chamber **14** to match the fuel delivery rate through the motorized needle valve **24** to correspond to the adjusted air mover throughput. In a related embodiment, a temperature sensor **70** may be used to generate and send a signal **71** indicating the temperature of the fluid leaving the fluid warming chamber **41**, either continuously or periodically, to the controller **50** for comparison to an operating set-point **61**. The controller **50** may be programmed to adjust the position of the motorized needle valve **24**, the throughput of the air mover **12**, or both, to bring the temperature of the fluid leaving the warming chamber **41** and the corresponding signal **71** close to the operating set-point **61.** Embodiments of the apparatus of the present invention having a system for enabling controller **50** monitoring and/or control of the position of the valve **24** that controls the rate of flow of fuel to the air-fuel mixing chamber **14** and/or the throughput of the air mover **12** can be used to optimize the air/fuel ratio within the catalyst compartment **25** and thereby conserve both fuel and battery life.

The interior surfaces of the gas chamber **39** and/or the fluid warming chamber **41** may include undulations, ridges, channels or other features that increase the overall surface area of the gas chamber **39** and/or the fluid warming chamber **41** to promote increased heat transfer from the first side **22A** of the heat exchanger base **16** to the second side **22B** of the heat exchanger base **16.** The interior surfaces of the fluid warming chamber cover **32** and the gas chamber cover **22** may be coated, treated and/or without undulations, ridges, channels or other features that increase the overall surface area of the fluid warming chamber cover **32** and the gas chamber cover **22** in order to minimize heat transfer from the gas chamber **39** to a component of the apparatus other than the heat exchanger base **16** across which heat is conducted to the fluid warming chamber **41**.

## Claims

1. An apparatus, comprising:
a gas flow chamber (39) on a first side of the apparatus having an air-fuel mixture inlet (23), a catalyst compartment (25) containing a catalyst member (20) and at least one tortuous combustion products pathway (18) for moving combustion products from the catalyst compartment to an exhaust gas port (19);
a fluid warming chamber (41) on a second side of the apparatus to conductively receive heat generated in the gas flow chamber (39) and having a fluid inlet (34) connectable to a source of fluid, a fluid warming surface and a fluid outlet (34) connectable to a patient;
an air-fuel mixing chamber (14) having an air inlet (21), a fuel port (17) and an air-fuel mixture outlet;
a motor-driven air mover (12) having an air intake to receive ambient air and an air outlet (37) disposed to discharge air to the air inlet (21) of the air-fuel mixing chamber (14);
a fuel assembly (45) comprising a fuel storage tank (13), a valve (24) to receive a stream of fuel from the tank and a fuel port connector (26) coupled to provide fuel from the valve to the fuel port (17) of the air-fuel mixing chamber (14);
a battery (36) to provide electrical current to operate a motor to drive the air mover (12); and
wherein a stream of an air-fuel mixture emerging from the air-fuel mixing chamber (14) enters the catalytic compartment (25) containing the catalyst member (20) and combusts to create a stream of heated combustion products;
wherein the combustion products flow through the at least one tortuous pathway (18) to the exhaust port (19) where the combustion products are liberated to the atmosphere; and
wherein a stream of fluid from the source of fluid enters the fluid warming chamber (41) through the fluid inlet (34), is warmed along the warming surface and is removed from the fluid warming chamber through the fluid outlet (34).

2. The apparatus of claim 1, wherein the catalyst member (20) comprises one of palladium and platinum.

3. The apparatus of claim 1, wherein the fuel stored in the tank (13) is a hydrocarbon.

4. The apparatus of claim 1, wherein the heat exchanger base comprises a metal alloy.

5. The apparatus of claim 4, wherein the heat exchanger base comprises aluminum.

6. The apparatus of claim 1, wherein the fluid warmed in the fluid warming chamber (41) is one of blood and intravenous fluid.

7. The apparatus of claim 1, further comprising:
a fuel cell (30) configured to receive a flow of fuel gas and to generate an electrical current to operate a motor within the air mover (12).

8. The apparatus of claim 1, wherein the valve (24) is adjustable to vary a rate of flow of fuel from the storage tank (13) to the air-fuel mixing chamber (14).

9. The apparatus of claim 1, wherein the warming surface of the fluid warming chamber (41) comprises an undulating surface to increase the surface area across which heat can be received from the gas chamber (39) and transferred to the fluid within the fluid warming chamber (41).

10. The apparatus of claim 1, further comprising:
a controller (50) coupled to receive a signal corresponding to an operating setpoint input (61) by a user of the apparatus;
wherein the controller generates and sends a signal to at least one of the motorized fuel valve (24) and the air mover (12) to adjust at least one of the rate of fuel and the rate of air delivered to the air-fuel mixing chamber (14).

## Patentansprüche

1. Vorrichtung, die Folgendes umfasst:
eine Gasströmungskammer (39) auf einer ersten Seite der Vorrichtung, die einen Luft-Brennstoffgemischeinlass (23), einen Katalysatorraum (25), der ein Katalysatorelement (20) enthält, und mindestens einen gewundenen Verbrennungsproduktpfad (18) zum Bewegen von Verbrennungsprodukten von dem Katalysatorraum zu einer Abgasöffnung (19) aufweist;
eine Fluidaufwärmkammer (41) auf einer zweiten Seite der Vorrichtung, um in der Gasströmungskammer (39) erzeugte Wärme leitend aufzunehmen, und die einen mit einer Quelle von Fluid verbindbaren Fluideinlass (34), eine Fluidaufwärmfläche und einen mit einem Patienten verbindbaren Fluidauslass (34) aufweist;
eine Luft-Brennstoffmischkammer (14), die einen Lufteinlass (21), eine Brennstofföffnung (17) und einen Luft-Brennstoffgemischauslass aufweist;
einen motorbetriebenen Lüfter (12), der einen Lufteintritt zum Aufnehmen von Umgebungsluft und einen Luftauslass (37), der angeordnet ist, um Luft zu dem Lufteinlass (21) der Luft-Brennstoffmischkammer (14) auszulassen, aufweist;
eine Brennstoffanordnung (45), umfassend einen Brennstoffspeicherbehälter (13), ein Ventil (24) zum Aufnehmen eines Stroms von Brennstoff von dem Behälter und einen Brennstofföffnungsverbinder (26), der gekoppelt ist, um Brennstoff von dem Ventil zu der Brennstofföffnung (17) der Luft-Brennstoffmischkammer (14) bereitzustellen;
eine Batterie (36), um elektrischen Strom zum Betreiben eines Motors zum Antreiben des Lüfters (12) bereitzustellen; und
wobei ein aus der Luft-Brennstoffmischkammer (14) austretender Strom eines Luft-Brennstoffgemischs in den das Katalysatorelement (20) enthaltenden Katalysatorraum (25) eintritt und verbrennt, um einen Strom erhitzter Verbrennungsprodukte zu erzeugen;
wobei die Verbrennungsprodukte durch den mindestens einen gewundenen Pfad (18) zu der Abgasöffnung (19) strömen, wo die Verbrennungsprodukte in die Atmosphäre freigesetzt werden; und
wobei ein Strom von Fluid von der Quelle von Fluid durch den Fluideinlass (34) in die Fluidaufwärmkammer (41) eintritt, entlang der Aufwärmfläche aufgewärmt wird und durch den Fluidauslass (34) aus der Fluidaufwärmkammer abgeführt wird.

2. Vorrichtung nach Anspruch 1, wobei das Katalysatorelement (20) Palladium oder Platin umfasst.

3. Vorrichtung nach Anspruch 1, wobei es sich bei dem in dem Behälter (13) gespeicherten Brennstoff um einen Kohlenwasserstoff handelt.

4. Vorrichtung nach Anspruch 1, wobei die Wärmetauscherbasis eine Metlalllegierung umfasst.

5. Vorrichtung nach Anspruch 4, wobei die Wärmetauscherbasis Aluminium umfasst.

6. Vorrichtung nach Anspruch 1, wobei es sich bei dem in der Fluidaufwärmkammer (41) aufgewärmten Fluid um Blut oder intravenöses Fluid handelt.

7. Vorrichtung nach Anspruch 1, die weiter Folgendes umfasst:
eine Brennstoffzelle (30), die dazu konfiguriert ist, einen Strom von Brenngas aufzunehmen und einen elektrischen Strom zu erzeugen, um einen Motor in dem Lüfter (12) zu betreiben.

8. Vorrichtung nach Anspruch 1, wobei das Ventil (24) verstellbar ist, um einen Volumenstrom von Brennstoff von dem Speicherbehälter (13) zu der Luft-Brennstoffmischkammer (14) zu ändern.

9. Vorrichtung nach Anspruch 1, wobei die Aufwärmfläche der Fluidaufwärmkammer (41) eine wellige Oberfläche umfasst, um den Oberflächenbereich zu vergrößern, über den Wärme von der Gaskammer (39) her aufgenommen und auf das Fluid in der Fluidaufwärmkammer (41) übertragen werden kann.

10. Vorrichtung nach Anspruch 1, die weiter Folgendes umfasst:
eine Steuereinheit (50), die gekoppelt ist, um ein Signal zu empfangen, das einer von einem Benutzer der Vorrichtung eingegebenen Betriebssollwerteingabe (61) entspricht;
wobei die Steuereinheit ein Signal erzeugt und zu dem motorisierten Brennstoffventil (24) und/oder dem Lüfter (12) sendet, um die Menge an Brennstoff und/oder die Menge an Luft, die in die Luft-Brennstoffmischkammer (14) zugeführt werden, zu verstellen.

## Revendications

1. Appareil, comprenant :
une chambre d'écoulement de gaz (39) sur un premier côté de l'appareil ayant une entrée de mélange air/combustible (23), un compartiment de catalyseur (25) contenant un élément de catalyseur (20) et au moins un trajet de produits de combustion tortueux (18) pour déplacer des produits de combustion du compartiment de catalyseur vers un orifice de gaz d'échappement (19) ;
une chambre de réchauffage de fluide (41) sur un second côté de l'appareil pour recevoir de façon conductrice une chaleur générée dans la chambre d'écoulement de gaz (39) et ayant une entrée de fluide (34) pouvant être raccordée à une source de fluide, une surface de réchauffage de fluide et une sortie de fluide (34) pouvant être raccordée à un patient ;
une chambre de mélange air/combustible (14) ayant une entrée d'air (21), un orifice de combustible (17) et une sortie de mélange air/combustible ;
un appareil aéraulique entraîné par moteur (12) présentant une entrée d'air pour recevoir de l'air ambiant et une sortie d'air (37) disposée pour décharger de l'air vers l'entrée d'air (21) de la chambre de mélange air/combustible (14) ;
un ensemble de combustible (45) comprenant un réservoir de stockage de combustible (13), une soupape (24) pour recevoir un courant de combustible à partir du réservoir et un raccord d'orifice de combustible (26) accouplé pour fournir du combustible de la soupape à l'orifice de combustible (17) de la chambre de mélange air/combustible (14) ;
une batterie (36) pour fournir un courant électrique pour actionner un moteur pour entraîner l'appareil aéraulique (12) ; et
dans lequel un courant d'un mélange air/combustible émergeant de la chambre de mélange air/combustible (14) entre dans le compartiment catalytique (25) contenant l'élément de catalyseur (20) et réalise une combustion pour créer un courant de produits de combustion chauffés ;
dans lequel les produits de combustion s'écoulent à travers l'au moins un trajet tortueux (18) vers l'orifice d'échappement (19) où les produits de combustion sont libérés dans l'atmosphère ; et
dans lequel un courant de fluide en provenance de la source de fluide entre dans la chambre de réchauffage de fluide (41) à travers l'entrée de fluide (34), est réchauffé le long de la surface de réchauffage et est retiré de la chambre de réchauffage de fluide à travers la sortie de fluide (34).

2. Appareil selon la revendication 1, dans lequel l'élément de catalyseur (20) comprend un élément parmi du palladium et du platine.

3. Appareil selon la revendication 1, dans lequel le combustible stocké dans le réservoir (13) est un hydrocarbure.

4. Appareil selon la revendication 1, dans lequel la base d'échangeur de chaleur comprend un alliage de métal.

5. Appareil selon la revendication 4, dans lequel la base d'échangeur de chaleur comprend de l'aluminium.

6. Appareil selon la revendication 1, dans lequel le fluide réchauffé dans la chambre de réchauffage de fluide (41) est un élément parmi du sang et un fluide intraveineux.

7. Appareil selon la revendication 1, comprenant en outre :
une pile à combustible (30) configurée pour recevoir un écoulement de gaz combustible et pour générer un courant électrique pour actionner un moteur à l'intérieur de l'appareil aéraulique (12).

8. Appareil selon la revendication 1, dans lequel la soupape (24) est réglable pour faire varier un débit de combustible du réservoir de stockage (13) à la chambre de mélange air/combustible (14).

9. Appareil selon la revendication 1, dans lequel la surface de réchauffage de la chambre de réchauffage de fluide (41) comprend une surface ondulatoire pour augmenter la surface active sur laquelle de la chaleur peut être reçue à partir de la chambre de gaz (39) et transférée vers le fluide à l'intérieur de la chambre de réchauffage de fluide (41).

10. Appareil selon la revendication 1, comprenant en outre :
un dispositif de commande (50) couplé pour recevoir un signal correspondant à une entrée de point de consigne de fonctionnement (61) par un utilisateur de l'appareil ;
dans lequel le dispositif de commande génère et envoie un signal à au moins un élément parmi la soupape de commande motorisée (24) et l'appareil aéraulique (12) pour régler au moins un élément parmi le débit de combustible et le débit d'air distribué à la chambre de mélange air/combustible (14).
